# FASCICULE DE BREVET EUROPEEN

(11) **EP 4 136 430 B1**
(45) Date de publication et mention de la délivrance du brevet: **27.12.2023**
(21) Numéro de dépôt: 21732487.0
(22) Date de dépôt: 26.05.2021
(51) Int. Cl.: G01N 21/552, G01N 27/414, G01N 33/543, G01N 33/94, G01N 33/569, C12Q 1/10

(54) **CAPTEUR BIOLOGIQUE ET/OU BIOCHIMIQUE ET/OU CHIMIQUE**
BIOLOGISCHER UND/ODER BIOCHEMISCHER UND/ODER CHEMISCHER SENSOR
BIOLOGICAL AND/OR BIOCHEMICAL AND/OR CHEMICAL SENSOR

(30) Priorité: 27.05.2020 FR 2005584
(43) Date de publication de la demande: 22.02.2023
(73) Titulaire: Commissariat à l'Energie Atomique et aux Energies Alternatives, 75015 Paris (FR); UNIVERSITÉ GRENOBLE ALPES, 38400 Saint-Martin-d'Heres (FR); CENTRE NATIONAL DE LA RECHERCHE SCIENTIFIQUE, 75794 Paris Cedex 16 (FR)
(72) Inventeur: ALAVA, Thomas, 38054 GRENOBLE CEDEX 09 (FR); MAILLEY, Pascal, 38054 GRENOBLE CEDEX 09 (FR); HOU-BROUTIN, Yanxia, 38850 BILIEU (FR); LEROY, Loïc, 38000 GRENOBLE (FR)
(74) Mandataire: Brevalex
(86) Numéro de dépôt international: PCT/FR2021/050951
(87) Numéro de publication internationale: WO 2021/240110

(56) Documents cités:
- US-A1- 2018 217 138
- US-A1- 2019 257 844
- SMITH CHRISTIAN W ET AL: "Wavelength-selective visible-light detector based on integrated graphene transistor and surface plasmon coupler", PROCEEDINGS OF SPIE, IEEE, US, vol. 9083, 4 juin 2014 (2014-06-04), pages 90832Q-90832Q, XP060037147, DOI: 10.1117/12.2050018 ISBN: 978-1-62841-730-2
- TAYLOR A D ET AL: "Quantitative and simultaneous detection of four foodborne bacterial pathogens with a multi-channel SPR sensor", BIOSENSORS AND BIOELECTRONICS, ELSEVIER SCIENCE LTD. UK, AMSTERDAM, NL, vol. 22, no. 5, 15 décembre 2006 (2006-12-15), pages 752-758, XP024961562, ISSN: 0956-5663, DOI: 10.1016/J.BIOS.2006.03.012 [extrait le 2006-12-15]

## Description

La présente invention se rapporte au domaine des capteurs, et plus particulièrement des capteurs biologiques et/ou biochimiques et/ou chimiques. Notamment, la présente invention concerne un capteur mettant en oeuvre la résonance de plasmons de surface et la caractérisation des propriétés électriques de transistors adaptés pour la détection d'espèces biologiques et/ou biochimiques et/ou chimiques.

### ÉTAT DE LA TECHNIQUE ANTÉRIEURE

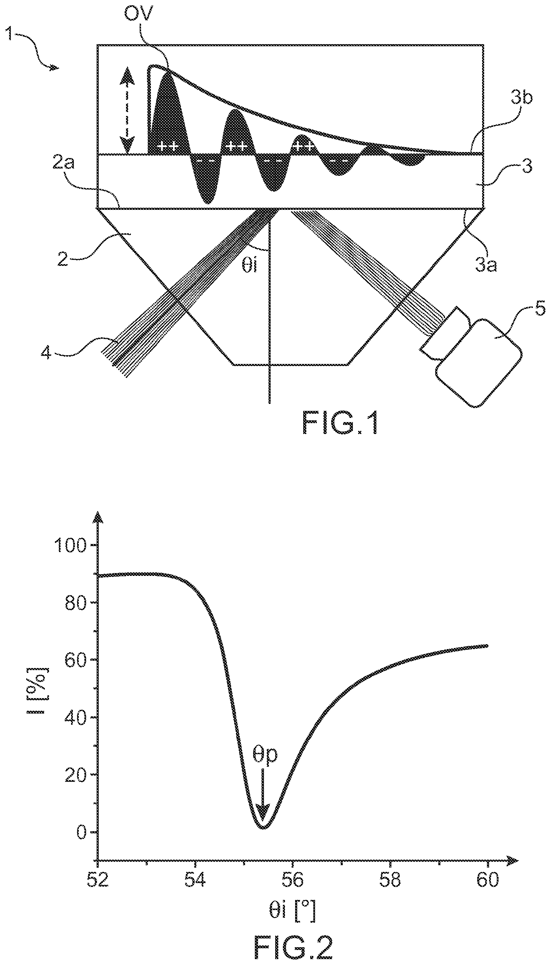

Un capteur 1 d'imagerie par résonance de plasmons de surface connu de la technique est illustré à la figure 1.

Ce dispositif 1 comprend notamment :
- un prisme 2 qui comprend une face, dite première face 2a ;
- une couche métallique 3 en recouvrement par une de ses faces, dite face de contact 3a, de la première face 2a ;
- une source d'émission lumineuse 4 destinée à émettre un rayonnement lumineux selon un angle d'incidence θi sur la face de contact 3a ;
- un détecteur 5, par exemple un détecteur CCD ou CMOS, destiné à collecter le rayonnement lumineux émis par la source d'émission lumineuse et réfléchi par la face de contact 3a.

En fonctionnement, la source d'émission lumineuse 4 émet un rayonnement lumineux en direction de la face de contact 3a selon un angle d'incidence θi variant, par exemple, de manière continue, dans une gamme d'angles. Ce rayonnement lumineux est alors réfléchi par la face de contact 3a pour être collecté par le détecteur 5.

Il est toutefois un angle d'incidence θp pour lequel l'énergie portée par le rayonnement lumineux incident crée une onde évanescente OV (représentée à la figure 1) susceptible d'entrer en résonance avec une onde électromagnétique de plasmons de surface de la couche métallique 3. Ce phénomène se traduit par une diminution de l'intensité du rayonnement lumineux réfléchi détectée au niveau du détecteur 5 tel qu'illustré à la figure 2.

Sur cette figure 2, on observe clairement une chute brutale de l'intensité relative du rayonnement lumineux réfléchie pour un angle d'incidence θi supérieur à 54°, et qui atteint son minimum pour l'angle d'incidence θp, autrement nommé angle de résonance plasmonique. Ce dernier dépend de la longueur d'onde du rayonnement lumineux, mais également de l'indice optique du milieu diélectrique au contact de la face exposée 3b de la couche métallique 3 et opposée à la face de contact 3a.

L'angle d'incidence θp, à la figure 2, dépend toutefois des propriétés optiques des matériaux mis en oeuvre et des caractéristiques, et notamment de la longueur d'onde, de la source d'émission lumineuse.

Il a été démontré que ce type de capteur, qui permet de détecter des variations extrêmement faibles de l'indice optique du milieu diélectrique, peut avantageusement être mis à profit pour la détection d'espèces biologiques, et/ou biochimiques et/ou chimiques (noté ci-après « cible »).

En effet, la simple adsorption de cibles au niveau de la face exposée 3b de la couche métallique 3 modifie localement l'indice optique de l'interface formée par la couche métallique 3 et le milieu diélectrique. La variation d'indice qui se traduit par une variation de l'angle de résonance plasmonique constitue ainsi un paramètre de choix pour la détection et/ou l'identification de cibles.

Afin de rendre l'analyse des cibles sélective, il peut être considéré de fonctionnaliser la face exposée 3b. Cette fonctionnalisation permet notamment de rendre l'adsorption au niveau de la face exposée 3b sélective à un type donné de cible, dite cible spécifique. En d'autres termes, la fonctionnalisation de la face exposée n'a d'autre but que de limiter le phénomène d'adsorption à la seule cible spécifique. Cette fonctionnalisation met généralement en oeuvre des espèces chimiques ou biochimiques ou biologiques (nommées ci-après « sondes ») greffées sur la face exposée 3b. À titre d'exemple, les sondes peuvent comprendre un aptamère au niveau duquel une cible formée par de l'adénosine est susceptible d'être adsorbée.

Ainsi, la mise en oeuvre d'une fonctionnalisation au moyen d'une sonde spécifique rend un tel capteur par résonance de plasmons de surface spécifique à une cible donnée, dite cible spécifique. En particulier, ce capteur spécifique peut être mis en oeuvre pour la détection de la cible spécifique contenue dans un liquide mouillant la face exposée 3b. Cette détection peut s'exercer jusqu'à une distance d'au moins 100 nm de la face exposée 3b.

Néanmoins, la sélectivité d'adsorption n'est pas parfaite de sorte que des phénomènes d'interférences sont susceptibles d'intervenir. Ces derniers sont notamment dus à l'adsorption, via une interaction non spécifique, de cibles non spécifiques présentes dans le fluide en cours d'analyse. Ce phénomène d'interférences dégrade la qualité de la détection, et peut, le cas échéant, être une source d'erreur dans l'analyse d'un fluide donné.

A titre d'exemple, un tel capteur peut être fonctionnalisé avec un aptamère jouant le rôle de sonde spécifique interagissant de manière spécifique (voire préférentielle) avec l'adénosine. Néanmoins, la sélectivité de cette interaction n'étant pas parfaite, la sonde spécifique peut également interagir avec des protéines autres que l'adénosine. Cette dernière interaction dégrade la qualité la détection et en fausse le résultat.

Par ailleurs, cette technique est souvent limitée à la détection de cibles de masse molaire importante et/ou de concentration relativement importantes.

De manière alternative, un capteur au graphène, et notamment un transistor fait de graphène, peut être utilisé pour la détection de la cible spécifique dans un liquide. Ce type de capteur, qui implique également une fonctionnalisation similaire à celle d'un capteur à résonance de plasmons de surface, est également sensible aux phénomènes d'interférences relatifs à la présence de cibles non spécifiques (en d'autres termes, autres que la cible spécifique).

Par ailleurs, un tel transistor, bien que relativement efficace pour la détection de cibles de faible masse molaire et/ou faible concentration, reste limité à la détection de ces dernières à quelques dizaines de nanomètres de la surface dudit transistor.

Par ailleurs, l'extrême sensibilité du graphène à son environnement limite le champ d'application à l'analyse de solutions peu complexes.

US 2019/257844 A1 présente un capteur biochimique qui prévoit une détection combinée par SPR avec un prisme et en utilisant un transistor. L'article Smith Christian et al. "Wavelength-selective visible-light detector based on integrated graphene transistor and surface plasmon coupler" vol. 9083, 4 juin 2014, présente egalement un capteur biochimique mais ne prévoit pas de functionnalisation du feuillet avec des sondes spécifiques.

Ainsi, un but de la présente invention est de proposer un capteur pour la détection et la reconnaissance d'espèces biologiques, et/ou biochimiques et/ou chimiques dont l'efficacité est améliorée.

Un autre but de la présente invention est de proposer un capteur permettant la détection et la reconnaissance de cibles avec une sensibilité améliorée.

### EXPOSÉ DE L'INVENTION

Les buts de l'invention sont, au moins en partie, atteints par un capteur biologique et/ou biochimique et/ou chimique selon la revendication 1.

Selon un mode de mise en oeuvre, la fonctionnalisation est également adaptée pour maintenir à une distance supérieure à la distance de détection Dd toute cible autre que la cible spécifique et susceptible de s'adsorber au niveau des sondes spécifiques, de sorte que seule la détection desdites cibles par résonance de plasmons de surface soit possible.

Selon un mode de mise en oeuvre, les sondes spécifiques sont des agents biologiques ou biochimiques ou chimiques greffés sur la face avant, chaque sonde spécifique étant adaptée pour que, lorsque la cible spécifique est adsorbée au niveau de la sonde spécifique, ladite sonde se déforme afin de placer ladite cible spécifique à une distance de la face avant inférieure à la distance de détection Dd.

Selon un mode de mise en oeuvre, le capteur comprend au moins un transistor de référence, qui comprend un feuillet de référence, fait du matériau bidimensionnel, et le feuillet de référence, destiné à former une région de canal, repose par une face arrière sur la deuxième couche, le feuillet de référence comprend également une face de référence et adaptée pour être le siège d'une adsorption des cibles de manière non sélective, de sorte que ces dernières puissent faire l'objet d'une détection lors d'une mesure combinée, au niveau de la face de référence, par résonnance de plasmons de surface initiés par la source lumineuse, et par mesure électrique au moyen du transistor de référence, la première couche formant avantageusement une électrode de grille de l'au moins un transistor de référence.

Selon un mode de mise en oeuvre, le dispositif comprend au moins un site, au niveau d'une section exposée à l'environnement extérieur de la première couche, dédié uniquement à la mesure par résonance de plasmons de surface.

Selon un mode de mise en oeuvre, le matériau à deux dimensions comprend au moins l'un des matériaux choisi parmi : graphène, sulfure de molybdène.

Selon un mode de mise en oeuvre, le matériau métallique comprend au moins l'un des éléments choisi parmi : or, aluminium, cuivre, argent.

Selon un mode de mise en oeuvre, ledit dispositif comprend en outre un capot en recouvrement par la première face, et dans lequel un fluide, circulant d'une voie d'entrée vers une voie de sortie dudit capot, est susceptible de faire l'objet d'une reconnaissance moléculaire au moyen dudit dispositif.

Selon un mode de mise en oeuvre, la source d'émission lumineuse et le détecteur sont agencés pour permettre d'exécuter des mesures à différents angles d'incidence θi.

Selon un mode de mise en oeuvre, la source d'émission lumineuse est polychromatique, et est agencée avec le détecteur pour que la mesure de résonance de plasmons de surface soit exécutée à angle fixe.

Selon un mode de mise en oeuvre, le détecteur est un détecteur matriciel.

Selon un mode de mise en oeuvre, la sonde spécifique est adaptée pour favoriser l'adsorption de bactéries Escherichia coli, et la reconnaissance d'une cible spécifique à cette famille.

Selon un mode de mise en oeuvre, la fonctionnalisation spécifique est adaptée pour favoriser l'adsorption des cibles de la famille de la cocaïne.

Selon un mode de mise en oeuvre, la fonctionnalisation spécifique (155) est adaptée pour favoriser l'adsorption de la thrombine α.

### BRÈVE DESCRIPTION DES DESSINS

D'autres caractéristiques et avantages apparaîtront dans la description qui va suivre d'un capteur biologique et/ou biochimique et/ou chimique selon l'invention, donnés à titre d'exemples non limitatifs, en référence aux dessins annexés dans lesquels :
La figure 1 est une représentation schématique selon un plan de coupe perpendiculaire à la première couche d'un dispositif de résonance de plasmons de surface connu de l'état de la technique ;
La figure 2 est une représentation graphique de l'intensité I relative du rayonnement lumineux réfléchie (axe vertical, en « % ») en fonction de l'angle d'incidence θi (axe horizontal, en « ° ») ;
La figure 3 est une représentation selon un plan de coupe d'un capteur selon la présente invention ;
La figure 4a est une représentation selon une vue en perspective du capteur biologique et/ou biochimique et/ou chimique selon la présente invention ;
La figure 4b est une illustration du mécanisme d'adsorption via l'interaction spécifique entre une cible spécifique et une sonde spécifique ;
La figure 4c est une illustration de l'effet de l'absorption de cibles autres que les cibles spécifiques au niveau des sondes spécifiques ;
Les figures 5a, 5b, 5c, 5d, 5e, 5f sont des représentations schématiques des différentes étapes de fabrication du capteur biologique et/ou biochimique et/ou chimique selon la présente invention.

### EXPOSÉ DÉTAILLÉ DE MODES DE RÉALISATION PARTICULIERS

La présente invention concerne un capteur biologique et/ou biochimique et/ou chimique, et notamment de reconnaissance d'espèces biologiques et/ou biochimiques et/ou chimiques, comme par exemple des bactéries Escherichia coli.

Le capteur selon la présente invention comprend notamment un site, dit site de reconnaissance, permettant de mettre en oeuvre une mesure combinée impliquant l'imagerie par résonance de plasmons de surface et une reconnaissance au moyen d'un transistor à effet de champ (ci-après « transistor »).

Cette mesure combinée sur un même site de reconnaissance permet notamment de mieux différencier des agents biologiques et/ou biochimiques et/ou chimiques susceptibles d'être détectées par l'une et/ou l'autre des deux techniques impliquées.

La suite de la description fait référence aux termes « sonde », et « cible ».

Par « cible », on entend une espèce biologique, ou biochimique ou chimique présente dans une solution, par exemple une solution liquide. Notamment, cette cible, selon la présente invention, est destinée à être détectée par un capteur décrit ci-après.

Par « sonde », on entend une espèce ou un agent biologique, ou biochimique ou chimique greffé sur une surface de manière à former une couche de fonctionnalisation sur ladite surface. Ces sondes peuvent notamment être adaptées pour favoriser l'adsorption des cibles sur la surface en question.

Par ailleurs et par définition selon la présente invention, l'adsorption d'une cible donnée, dite cible spécifique, au niveau de sondes données, dites sondes spécifiques, peut donner lieu à une interaction particulière. Cette interaction, dite interaction spécifique, en plus du phénomène d'adsorption, peut donner lieu à une déformation structurelle de la sonde. Cette déformation peut notamment impliquer un repliement de la sonde spécifique de manière à rapprocher la cible spécifique de la surface. Il est entendu que l'interaction entre une sonde spécifique et toute cible, autre que la cible spécifique, se limitera à une simple adsorption de la cible en question.

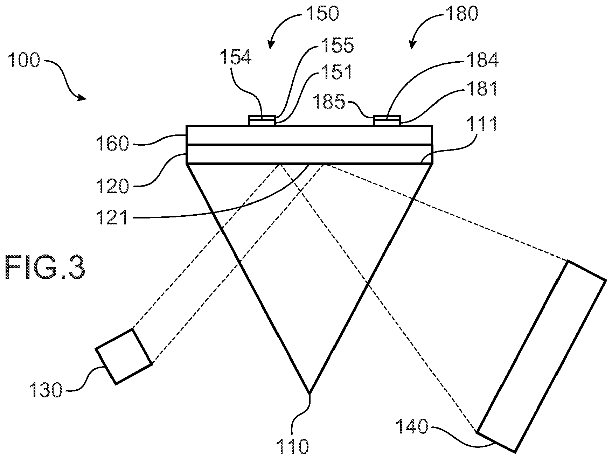

La figure 3 est une représentation schématique d'un capteur biologique et/ou biochimique et/ou chimique 100 (ci-après « capteur 100 ») selon la présente invention.

Le capteur 100 comprend notamment un support 110.

Ce support 110 est notamment transparent au rayonnement lumineux émis par une source d'émission lumineuse 130 décrite ci-après. Ce support 110 peut comprendre une lame de verre, ou un prisme.

La suite de la description et les figures ne font référence qu'au seul prisme sans toutefois limiter l'invention à ce seul aspect.

Le prisme 110 est pourvu d'une première face 111. Le prisme 110 peut notamment comprendre du verre.

Une couche, dite première couche 120, repose par une de ses faces, dite face de contact 121, sur la première face 111. La première couche 120 comprend un matériau métallique, et plus particulièrement au moins un des matériaux choisis parmi : or, aluminium, cuivre, argent.

Une couche d'adhésion peut par ailleurs être intercalée ente la première face et la première couche 120. Cette première couche peut notamment comprendre du Ni ou du Co, d'une épaisseur, par exemple, comprise entre 2 nm et 3 nm.

La première couche 120 peut présenter une épaisseur E comprise entre 15 nm et 100 nm.

Le capteur 100 comprend en outre une source d'émission lumineuse 130 destinée à émettre un rayonnement lumineux selon un angle d'incidence θi sur la face de contact 121.

Plus particulièrement et selon une première variante, la source d'émission lumineuse 130 peut être agencée de sorte que l'angle d'incidence θi varie, notamment de manière continue, dans une gamme d'angles d'incidence, par exemple au-delà de l'angle de réflexion totale. Le rayonnement lumineux susceptible d'être émis par la source de rayonnement lumineux est avantageusement non cohérent. A cet égard, la source d'émission lumineuse 130 peut comprendre une diode électroluminescente émettant un rayonnement visible, par exemple rouge.

Selon une deuxième variante, la source d'émission lumineuse 130 est polychromatique et émet un rayonnement lumineux à un angle fixe.

Le capteur 100 comprend également un détecteur 140.

Le détecteur 140 peut comprendre un ou plusieurs détecteurs ponctuels. Selon un autre aspect, le détecteur 140 peut être matriciel.

Par « détecteur matriciel », on entend un détecteur pourvu d'une pluralité de cellules de détection agencées selon un maillage des lignes et de colonnes. Plus particulièrement, les cellules de détection sont agencées à l'intersection des lignes et des colonnes.

La détecteur matriciel 140 peut par exemple comprendre un détecteur de type CCD ou CMOS.

Le détecteur 140 est par ailleurs agencé pour collecter le rayonnement lumineux émis par la source d'émission lumineuse 130 et réfléchi par la face de contact 121.

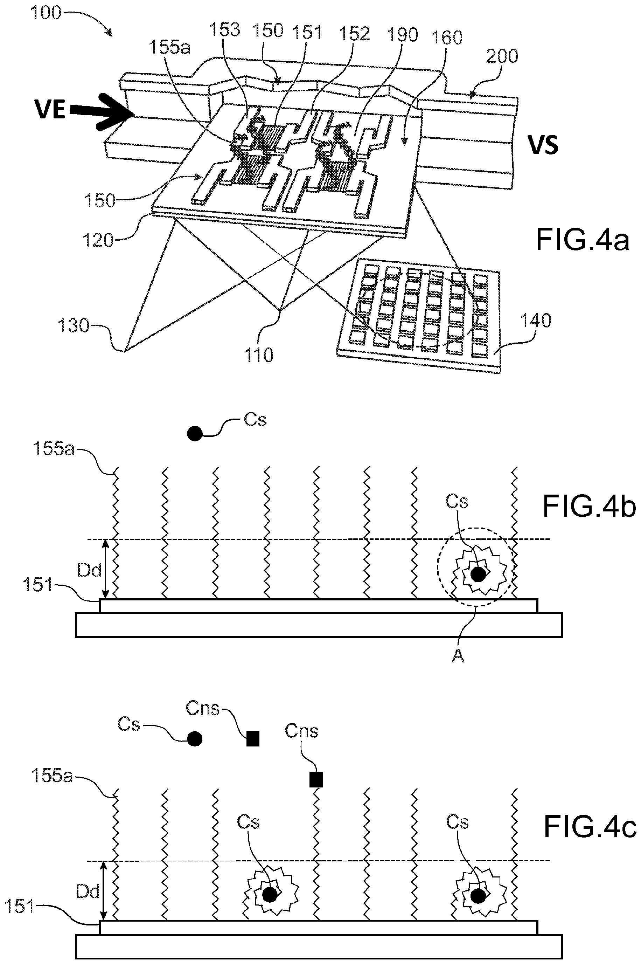

Le capteur 100 selon la présente invention comprend en outre au moins un transistor 150, et une deuxième couche 160, faite d'un matériau diélectrique s'interposant entre la première couche 120 et l'au moins un transistor 150 (figures 3 et 4a).

Le matériau diélectrique peut comprendre au moins un des matériaux choisi parmi : nitrure de silicium, oxyde de silicium, oxyde d'hafnium.

Notamment, le transistor 150 comprend un feuillet 151 fait d'un matériau bidimensionnel, reposant par sa face arrière sur la deuxième couche 160 et est destiné à former une région de canal du transistor 150.

Par « matériau bidimensionnel », on entend un matériau qui présente une structure cristalline à 2 dimensions. De tels matériaux comprennent en général un empilement de plans cristallins au sein desquels les atomes ou molécules sont liés par des liaisons covalentes, tandis des interactions du type Van der Walls assurent la cohésion entre plans cristallins.

De manière avantageuse, le matériau bidimensionnel est optiquement transparent au rayonnement lumineux susceptible d'être émis par la source d'émission lumineuse.

Par « optiquement transparent », on entend un coefficient de transmission supérieur à 70%.

Ainsi, le matériau bidimensionnel peut comprendre au moins un des matériaux choisis parmi : graphène, sulfure de molybdène.

Le transistor 150 comprend également deux électrodes dites, respectivement, électrode de source 152 et électrode de drain 153.

Le feuillet 151 comprend également une face opposée à la face arrière dudit feuillet, dite face avant 154, qui comprend une fonctionnalisation spécifique 155. Cette fonctionnalisation spécifique 155 comprend notamment des sondes spécifiques 155a au niveau desquelles des cibles spécifiques, d'une famille de cibles, sont susceptibles d'être adsorbées (figure 4a).

Notamment, la fonctionnalisation spécifique 155 est adaptée pour placer à une distance inférieure à une distance de détection Dd les cibles spécifiques susceptibles d'être adsorbées au niveau des sondes spécifiques. La distance de détection est notamment une distance en-dessous de laquelle la détection combinée par mesure électrique au moyen du transistor 150 et par mesure de résonance de plasmons de surface est possible.

La figure 4b est une illustration de mécanisme d'adsorption et de l'interaction spécifique décrite à titre liminaire de la présente section. La figure 4b représente notamment le feuillet 151 sur lequel sont greffées de sondes spécifiques 155a. En particulier, sur cette figure 4b, l'interaction spécifique (zone A de la figure 4b) d'une cible spécifique Cs avec une des sondes spécifiques 155a conduit au repliement de ladite sonde sur elle-même de sorte à rapprocher la cible en question une distance inférieure à la distance Dd.

A contrario, la fonctionnalisation spécifique 155 peut être adaptée pour maintenir à une distance supérieure à la distance de détection Dd toute cible, autre que la cible spécifique et susceptible de s'adsorber au niveau des sondes spécifiques. Ainsi, selon cet aspect, seule la détection desdites cibles (autres que la cible spécifique) par résonance de plasmons de surface est possible.

La figure 4c représente à cet égard l'effet de l'adsorption de cible autres que les cibles spécifiques, dites cibles non spécifiques Cns. Dans la mesure où, selon cet aspect, l'interaction spécifique n'intervient pas entres les sondes spécifiques et les cibles non spécifiques Cns, la déformation et/ou le repliement desdites sondes 155a n'est pas observé. De cette manière, ces cibles non spécifiques restent à distance de la face avant, et notamment à une distance supérieure à la distance Dd.

De manière avantageuse, la sonde spécifique peut comprendre un agent biologique, et notamment un aptamère par exemple pour la détection d'adénosine.

Ainsi, un tel capteur 100 peut avantageusement être mis en oeuvre pour l'analyse d'une solution complexe comprenant différentes cibles d'une famille de cibles spécifiques.

En particulier, la considération de la fonctionnalisation spécifique 155 selon les termes de la présente invention, à savoir une fonctionnalisation spécifique 155 permettant de localiser exclusivement la cible spécifique dans le champ de détection du transistor permet de confirmer la présence de ladite cible, également détectée par résonance de plasmons de surface. Les sondes spécifiques sont par ailleurs adaptées pour maintenir à distance de détection par le transistor toute cible autre que la cible spécifique.

En d'autres termes, le capteur 100 tel qu'énoncé, permet d'exacerber les avantages de deux sondes présentant des champs de détection, des sensibilités et des sélectivités différentes.

En effet, la détection par résonance de plasmons de surface permet de détecter, avec une grande sélectivité la présence des cibles spécifiques sur un champ de détection qui peut s'étendre sur une centaine de nanomètres à partir de la face avant 154. Le transistor 150 est pour sa part très sensible à la présence ou non de la cible spécifique dans son champ de détection qui peut s'étendre quelques dizaines de nanomètres, notamment 20 nm, à partir de la face avant 154. En outre, le maintien hors de ce champ de détection des cibles autres que la cible spécifique annihile toute détection de ces cibles autres que la cible spécifique par le transistor 150.

Le dispositif 100 selon la présente invention peut également comprendre au moins transistor de référence 180. Ce dernier comprend à cet égard un feuillet de référence 181, fait du matériau bidimensionnel, et destiné à former une région de canal.

Le transistor de référence 180 comprend également deux électrodes dites, respectivement, électrode de source 182 et électrode de drain 183.

Le feuillet de référence 181 repose par une face arrière sur la deuxième couche 160 et comprend également une face de référence 174.

Ladite face de référence est adaptée pour être le siège d'une adsorption des cible indifféremment de leur type, de sorte que ces dernières puissent faire l'objet d'une détection lors d'une mesure combinée, au niveau de la face de référence, par résonnance de plasmons de surface initiés par la source lumineuse, et par mesure électrique au moyen du transistor de référence 180.

Ce site de référence permet de connaître la réponse du capteur 100 en l'absence de fonctionnalisation, et par voie de conséquence faire des mesures différentielles.

Le capteur 100 peut également comprendre au moins un site 190, au niveau d'une section exposée à l'environnement extérieur de la première couche, dédié uniquement à la mesure par résonance de plasmons de surface. Ce site permet notamment de procéder à des mesures de référence et de calibration (figure 4a).

Le capteur 100 peut par ailleurs être pourvu d'un capot 200 (vue en coupe sur la figure 4a) en recouvrement par la première face, et dans lequel un fluide, circulant d'une voie d'entrée VE vers une voie de sortie VS dudit capot, est susceptible de faire l'objet d'une reconnaissance moléculaire au moyen dudit dispositif.

La première couche 120 essentiellement considérée pour la génération de plasmons de surface, peut également former des électrodes de grilles des différents transistors décrits dans la présente invention. En particulier, cette première couche peut être structurée afin d'adresser soit de manière individuelle, ou par groupes les différents transistors. Ce dernier aspect permet de simplifier le dispositif et notamment son procédé de fabrication.

Le capteur 100 selon la présente invention n'est pas limité à la mise en oeuvre d'un seul transistor par type de transistor. Il peut à cet égard être envisagé de mettre en oeuvre une pluralité de transistors spécifiques et de références. Un traitement informatique peut alors être mis en oeuvre pour le traitement des données collectées au niveau du détecteur 140.

Par ailleurs, le capteur 100 selon la présente invention, du fait d'une sensibilité et d'une sélectivité accrues, ouvre la voie à la détection de cibles de petite taille (et notamment d'une taille inférieure à 2 nm) et/ou peu concentrées et/ou de faible masse molaire.

Il est possible selon un premier exemple de détecter des traces de drogue et notamment des traces de cocaïne prise comme cible spécifique. Cette cible spécifique peut notamment interagir avec une sonde spécifique faite d'un aptamère du type MNS-4.1 tel que décrit dans le document [1] cité à la fin de la description.

Selon un deuxième exemple, la sonde spécifique peut comprendre un aptamère thrombin pour la détection de la thrombine α, tel que décrit dans le document [2] cité à la fin de la description.

De manière générale, le capteur 100 peut comprendre plusieurs transistors fonctionnalisés chacun avec une sonde spécifique différente. Ces différentes sondes spécifiques ouvrent la voie l'analyse de fluides complexes comprenant différents types de cibles spécifiques.

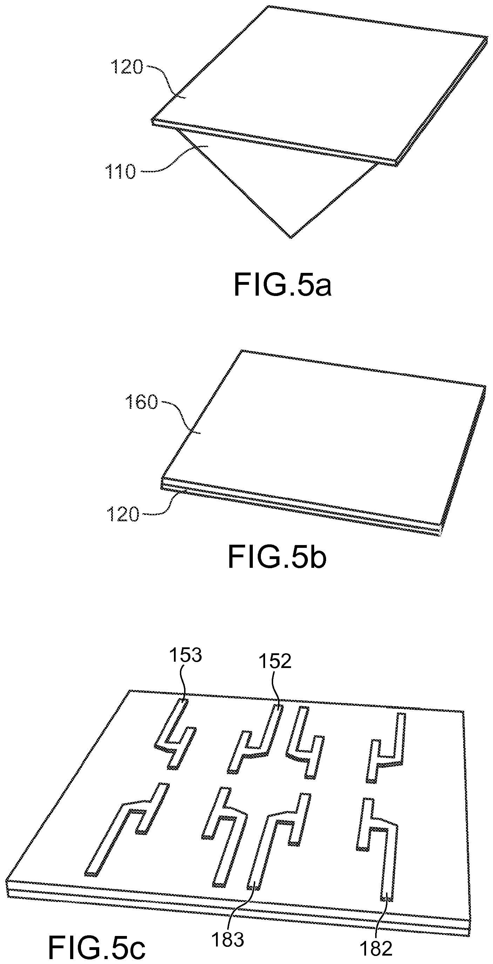

Le procédé de fabrication d'un tel capteur est décrit en relation avec les figures 5a à 5f.

Notamment, la figure 5a représente une étape de formation de la première couche 120, notamment en or, sur une première face d'un prisme.

Tel que représenté à la figure 5b, la deuxième couche 160, notamment en nitrure de silicium, est formée en recouvrement de la première couche 120.

La figure 5c est une représentation d'une étape de formation des contacts, et notamment des électrodes de source et de drain des différents transistors. Cette étape implique plus particulièrement la formation d'une couche de titane, et une couche d'or, suivie de la définition de motif relatif auxdites électrode par une séquence de photolithographie / gravure (notamment par voie humide).

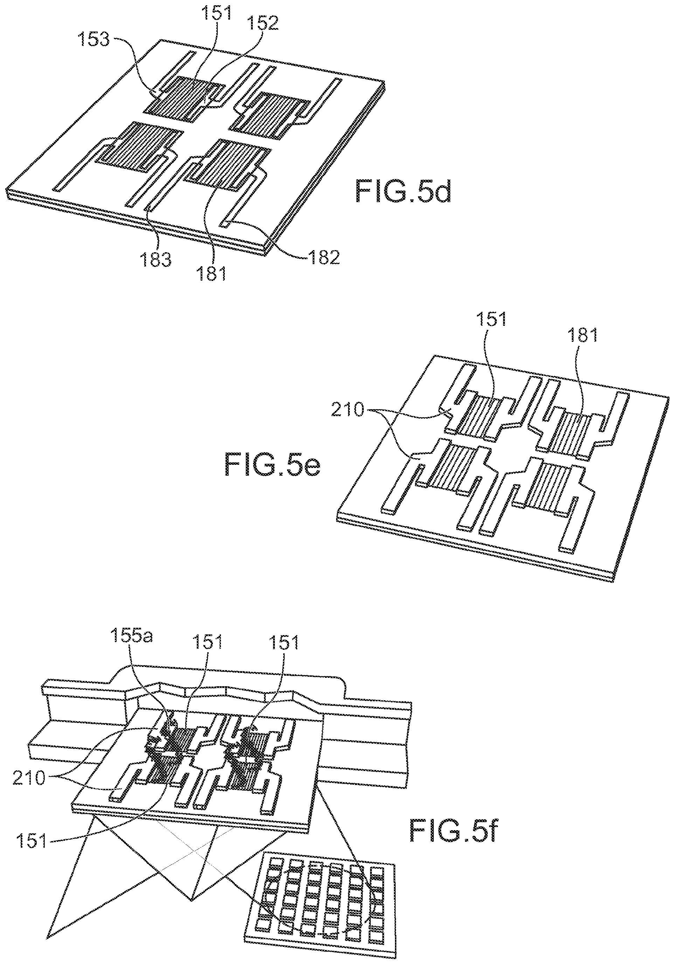

Une monocouche de graphène est ensuite transférée sur la deuxième couche 160 selon un procédé de transfert en voie liquide (technique dite du « fishing ») et mise en forme afin de prévenir toute diaphonie entre les transistors (figure 5d).

Une couche de passivation électrique 210 est ensuite formée sur les électrodes de source et de drain afin de protéger ces dernières. La couche de passivation électrique peut notamment impliquer la mise en oeuvre d'une résine du type SU8 (figure 5e).

Une dernière étape, représentée à la figure 5f, de fonctionnalisation biologique peut être exécutée.

### RÉFÉRENCES

[1] Milan N. Stojanivic et al., « Aptamer-Based Folding Fluorescent Sensor for Cocaine », J. Am. Chem. Soc., 123, 4928-4931, 2001;
[2] Lisa R. Paborsky et al., « The Single-standred DNA Aptamer-binding Site of Human Thrombin », The Journal of Biological Chemistry, 28(5), 20808-20811, 1993.

## Revendications

1. Capteur biologique et/ou biochimique et/ou chimique (100) comprenant :
- un prisme (110) qui comprend une face, dite première face (111) ;
- une première couche (120) métallique en recouvrement par une face, dite face de contact (121), de la première face (111) ;
- une source d'émission lumineuse (130) destinée à émettre un rayonnement lumineux selon un angle d'incidence θi sur la face de contact ;
- un détecteur (140) destiné à collecter le rayonnement lumineux émis par la source d'émission lumineuse et réfléchi par la face de contact (121) ;
le dispositif comprend une deuxième couche (160) de matériau diélectrique en recouvrement de la première couche (120), et sur laquelle repose un transistor (150) à effet de champ, ledit transistor étant doté d'une électrode de source (152) et d'une électrode de drain (153) comprend un feuillet (151), fait d'un matériau bidimensionnel présentant une structure cristalline à deux dimensions, le matériau bidimensionnel étant destiné à former une région de canal et reposant par une face arrière sur la deuxième couche, une face avant (154) du feuillet, opposée à sa face arrière, comprend une fonctionnalisation (155) avec des sondes spécifiques au niveau desquelles des cibles spécifiques, d'une famille de cibles, sont susceptibles d'être adsorbées, la fonctionnalisation spécifique (155) étant adaptée pour placer les cibles spécifiques adsorbées à une distance inférieure à une distance de détection Dd en-dessous de laquelle la détection combinée par mesure électrique au moyen du transistor spécifique (150) et par mesure de résonance de plasmons de surface est possible.

2. Capteur selon la revendication 1, dans lequel la fonctionnalisation spécifique (155) est également adaptée pour maintenir à une distance supérieure à la distance de détection Dd toute cible, autre que la cible spécifique et susceptible de s'adsorber au niveau des sondes spécifiques, de sorte que seule la détection desdites cibles par résonance de plasmons de surface soit possible.

3. Capteur selon la revendication 1 ou 2, dans lequel les sondes spécifiques sont des agents biologiques ou biochimiques ou chimiques greffés sur la face avant (154), chaque sonde spécifique étant adaptée pour que, lorsque la cible spécifique est adsorbée au niveau de la sonde spécifique, ladite sonde se déforme afin de placer ladite cible spécifique à une distance de face avant (154) inférieure à la distance de détection Dd.

4. Capteur selon l'une des revendications 1 à 3, dans lequel le capteur (100) comprend au moins un transistor de référence (180), qui comprend un feuillet de référence, fait du matériau bidimensionnel, et le feuillet de référence, destiné à former une région de canal, repose par une face arrière sur la deuxième couche, le feuillet de référence comprend également une face de référence et adaptée pour être le siège d'une adsorption des cibles de manière non sélective, de sorte que ces dernières puissent faire l'objet d'une détection lors d'une mesure combinée, au niveau de la face de référence, par résonnance de plasmons de surface initiés par la source lumineuse, et par mesure électrique au moyen du transistor de référence (180), la première couche formant avantageusement une électrode de grille de l'au moins un transistor de référence (180).

5. Capteur selon l'une des revendications 1 à 4, dans lequel le dispositif comprend au moins un site (190), au niveau d'une section exposée à l'environnement extérieur de la première couche, dédié uniquement à la mesure par résonance de plasmons de surface.

6. Capteur selon l'une des revendications 1 à 5, dans lequel le matériau à deux dimension comprend au moins l'un des matériaux choisi parmi : graphène, sulfure de molybdène.

7. Capteur selon l'une des revendications 1 à 6, dans lequel le matériau métallique comprend au moins l'un des éléments choisi parmi : or, aluminium, cuivre, argent.

8. Capteur selon l'une des revendications 1 à 7, dans lequel ledit dispositif comprend en outre un capot (200) en recouvrement par la première face, et dans lequel un fluide, circulant d'une voie d'entrée vers une voie de sortie dudit capot (200), est susceptible de faire l'objet d'une reconnaissance moléculaire au moyen dudit dispositif.

9. Capteur selon l'une des revendications 1 à 8, dans lequel la source d'émission lumineuse et le détecteur sont agencés pour permettre d'exécuter des mesures à différents angles d'incidence θi.

10. Capteur selon l'une des revendications 1 à 8, dans lequel la source d'émission lumineuse est polychromatique, et est agencée avec le détecteur pour que la mesure de résonance de plasmons de surface soit exécutée à angle fixe.

11. Capteur selon l'une des revendications 1 à 10, dans lequel le détecteur est un détecteur matriciel.

12. Capteur selon l'une des revendications 1 à 11, dans lequel la fonctionnalisation spécifique (155) est adaptée pour favoriser l'adsorption des cible de la famille de bactéries Escherichia coli, et la reconnaissance d'une cible spécifique à cette famille.

13. Capteur selon les revendications 1 à 11, dans lequel la fonctionnalisation spécifique (155) est adaptée pour favoriser l'adsorption des cibles de la famille de la cocaïne.

14. Capteur selon les revendications 1 à 11, dans lequel la fonctionnalisation spécifique (155) est adaptée pour favoriser l'adsorption de la thrombine α.

## Patentansprüche

1. Biologischer und/oder biochemischer und/oder chemischer Sensor (100), Folgendes umfassend:
- ein Prisma (110), das eine Fläche, genannt erste Fläche (111), umfasst;
- eine erste Metallschicht (120), die an einer Fläche, genannt Kontaktfläche (121), von der ersten Fläche (111) überdeckt wird,
- eine Lichtemissionsquelle (130), die dazu bestimmt ist, eine Lichtstrahlung gemäß einem Einfallswinkel θi auf die Kontaktfläche zu emittieren;
- einen Detektor (140), dazu bestimmt ist, die von der Lichtemissionsquelle emittierte und von der Kontaktfläche (121) reflektierte Lichtstrahlung zu sammeln;
wobei die Vorrichtung eine zweite Schicht (160) aus dielektrischem Material umfasst, die die erste Schicht (120) überdeckt und auf der ein Feldeffekttransistor (150) ruht, wobei der Transistor, der mit einer Source-Elektrode (152) und einer Drain-Elektrode (153) versehen ist, ein Blatt (151) umfasst, das aus einem zweidimensionalen Material hergestellt ist, das eine Kristallstruktur mit zwei Dimensionen aufweist, wobei das zweidimensionale Material dazu bestimmt ist, einen Kanalbereich zu bilden, und mit einer Rückseite auf der zweiten Schicht ruht, wobei eine Vorderseite (154) des Blattes, die der Rückseite gegenüberliegt, eine Funktionalisierung (155) mit spezifischen Sonden umfasst, an denen spezifische Ziele aus einer Familie von Zielen adsorbiert werden können, wobei die spezifische Funktionalisierung (155) so beschaffen ist, dass sie die adsorbierten spezifischen Ziele in einer Distanz platziert, die geringer ist als eine Detektionsdistanz Dd, unterhalb derer eine kombinierte Detektion durch elektrische Messung mittels des spezifischen Transistors (150) und durch Oberflächenplasmonenresonanzmessung möglich ist.

2. Sensor nach Anspruch 1, wobei die spezifische Funktionalisierung (155) ebenfalls so beschaffen ist, dass sie jedes andere Ziel als das spezifische Ziel, das an den spezifischen Sonden adsorbiert werden kann, in einer Distanz hält, die größer ist als die Detektionsdistanz Dd, so dass nur die Detektion dieser Ziele durch Oberflächenplasmonenresonanz möglich ist.

3. Sensor nach Anspruch 1 oder 2, wobei die spezifischen Sonden biologische oder biochemische oder chemische Mittel sind, die auf die Vorderseite (154) aufgepfropft sind, wobei jede spezifische Sonde so angepasst ist, dass, wenn das spezifische Ziel an der spezifischen Sonde adsorbiert wird, sich die Sonde verformt, um das spezifische Ziel in einer Distanz von der Vorderseite (154) zu platzieren, die geringer ist als die Detektionsdistanz Dd.

4. Sensor nach einem der Ansprüche 1 bis 3, wobei der Sensor (100) mindestens einen Referenztransistor (180) umfasst, der ein Referenzblatt umfasst, das aus dem zweidimensionalen Material hergestellt ist, und das Referenzblatt, das dazu bestimmt ist, einen Kanalbereich zu bilden, mit einer Rückseite auf der zweiten Schicht ruht, wobei das Referenzblatt ebenfalls eine Referenzfläche umfasst und so beschaffen ist, dass es eine Adsorption der Ziele auf nicht selektive Weise beherbergt, sodass diese bei einer kombinierten Messung auf der Ebene der Referenzfläche detektiert werden können, durch Resonanz von Oberflächenplasmonen, initiiert durch die Lichtquelle, und durch elektrische Messung mittels des Referenztransistors (180), wobei die erste Schicht vorteilhafterweise eine Gate-Elektrode des mindestens einen Referenztransistors (180) bildet.

5. Sensor nach einem der Ansprüche 1 bis 4, wobei die Vorrichtung mindestens eine Stelle (190) an einem Abschnitt, der der äußeren Umgebung der ersten Schicht ausgesetzt ist, umfasst, die ausschließlich der Resonanzmessung von Oberflächenplasmonen gewidmet ist.

6. Sensor nach einem der Ansprüche 1 bis 5, wobei das zweidimensionale Material mindestens eines der folgenden Materialien umfasst: Graphen, Molybdänsulfid.

7. Sensor nach einem der Ansprüche 1 bis 6, wobei das metallische Material mindestens eines der Elemente umfasst, die ausgewählt sind aus: Gold, Aluminium, Kupfer, Silber.

8. Sensor nach einem der Ansprüche 1 bis 7, wobei die Vorrichtung außerdem eine Haube (200) umfasst, die von der ersten Fläche überdeckt wird, und wobei ein Fluid, das von einem Einlassweg zu einem Auslassweg der Haube (200) fließt, mittels der Vorrichtung molekular erkannt werden kann.

9. Sensor nach einem der Ansprüche 1 bis 8, wobei die Lichtemissionsquelle und der Detektor so angeordnet sind, dass Messungen bei verschiedenen Einfallswinkeln θi durchgeführt werden können.

10. Sensor nach einem der Ansprüche 1 bis 8, wobei die Lichtemissionsquelle polychromatisch ist und mit dem Detektor so angeordnet ist, dass die Oberflächenplasmonenresonanzmessung unter einem festen Winkel durchgeführt wird.

11. Sensor nach einem der Ansprüche 1 bis 10, wobei der Detektor ein Matrixdetektor ist.

12. Sensor nach einem der Ansprüche 1 bis 11, wobei die spezifische Funktionalisierung (155) so angepasst ist, dass sie die Adsorption von Zielen aus der Bakterienfamilie Escherichia coli und die Erkennung eines für diese Familie spezifischen Ziels fördert.

13. Sensor nach Anspruch 1 bis 11, wobei die spezifische Funktionalisierung (155) so angepasst ist, dass sie die Adsorption von Zielen aus der Familie des Kokains fördert.

14. Sensor nach Anspruch 1 bis 11, wobei die spezifische Funktionalisierung (155) so angepasst ist, dass sie die Adsorption von Thrombin α fördert.

## Claims

1. Biological and/or biochemical and/or chemical sensor (100) comprising:
- a prism (110) which comprises a face, referred to as the first face (111);
- a first metal layer (120) covering a face, referred to as the contact face (121), of the first face (111);
- a light source (130) intended to emit light radiation at an angle of incidence θi on the contact face;
- a detector (140) intended to collect the light radiation emitted by the light source and reflected by the contact face (121);
the device comprises a second layer (160) of dielectric material covering the first layer (120), and on which a transistor (150) rests, said transistor comprises a sheet (151), made of a two-dimensional material, intended to form a channel region and resting with a rear face on the second layer, a front face (154) of the sheet, opposite its rear face, comprises a functionalisation (155) with specific probes at which specific targets of a target family are capable of being adsorbed, the specific functionalisation (155) being adapted to place the adsorbed specific targets at a distance lower than a detection distance Dd, below which the combined detection by electrical measurement by means of the specific transistor (150) and by surface plasmon resonance measurement is possible.

2. Sensor according to claim 1, wherein the specific functionalisation (155) is also adapted to maintain any target, other than the specific target and susceptible to adsorption at the specific probes, at a distance greater than the detection distance Dd, such that only the detection of said targets by surface plasmon resonance is possible.

3. Sensor according to claim 1 or 2, wherein the specific probes are biological or biochemical or chemical agents grafted onto the front face (154), each specific probe being adapted so that when the specific target is adsorbed at the specific probe, said probe deforms in order to place said specific target at a distance from the front face (154) lower than the detection distance Dd.

4. Sensor according to any of claims 1 to 3, wherein the sensor (100) comprises at least one reference transistor (180), which comprises a reference sheet, made of two-dimensional material, and the reference sheet, intended to form a channel region, rests with a rear face on the second layer, the reference sheet also comprises a reference face and is adapted to be the seat of adsorption of targets non-selectively, so that the latter can be detected in a combined measurement, at the level of the reference face, by resonance of surface plasmons initiated by the light source, and by electrical measurement by means of the reference transistor (180), the first layer forming advantageously a gate electrode of the at least one reference transistor (180).

5. Sensor according to any of claims 1 to 4, wherein the device comprises at least one site (190), at a section exposed to the external environment of the first layer, dedicated solely to surface plasmon resonance measurement.

6. Sensor according to any of claims 1 to 5, wherein the two-dimensional material comprises at least one of the materials selected from: graphene, molybdenum sulphide.

7. Sensor according to any of claims 1 to 6, wherein the metal material comprises at least one element selected from: gold, aluminium, copper, silver.

8. Sensor according to any of claims 1 to 7, wherein said device further comprises a cover (200) covering with the first face, and wherein a fluid, flowing from an inlet path to an outlet path of said cover (200), is capable of molecular recognition by means of said device.

9. Sensor according to any of claims 1 to 8, wherein the light source and the detector are arranged to enable measurements to be made at different angles of incidence θi.

10. Sensor according to any of claims 1 to 8, wherein the light source is polychromatic, and is arranged with the detector so that the surface plasmon reference measurement is performed at a fixed angle.

11. Sensor according to any of claims 1 to 10, wherein the detector is a matrix detector.

12. Sensor according to any of claims 1 to 11, wherein the specific functionalisation (155) is adapted to promote the adsorption of targets of the Escherichia coli family of bacteria, and recognition of a target specific to that family.

13. Sensor according to claims 1 to 11, wherein the specific functionalisation (155) is adapted to promote the adsorption of targets of the cocaine family.

14. Sensor according to claims 1 to 11, wherein the specific functionalisation (155) is adapted to promote the adsorption of α-thrombin.
